# EUROPEAN PATENT APPLICATION

(11) **EP 0 544 981 A2**
(43) Date of publication of application: **09.06.1993**
(21) Application number: 92110090.5
(22) Date of filing: 16.06.1992
(51) Int. Cl.: C07D 471/04, A61K 31/435

(54) **Naphthyridone carboxylic acid derivatives and antibacterial compositions containing them**

(30) Priority: 25.11.1991 US 798296; 07.04.1992 US 862152
(71) Applicant: WARNER-LAMBERT COMPANY, Morris Plains New Jersey 07950 (US)
(72) Inventor: Domagala, John Michael, Canton, Michigan 48188 (US); Hagen, Susan Elizabeth, Canton, Michigan 48187 (US); Kiely, John Steven, Ann Arbor, Michigan 48103 (US)
(74) Representative: Mansmann, Ivo

(57) **Abstract**

The application comprises new compounds of the formula IV 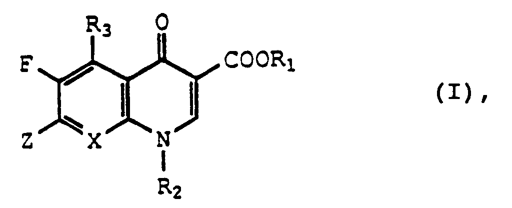
wherein R₂ is cyclopropyl or 2,4-difluorophenyl and Z is a group 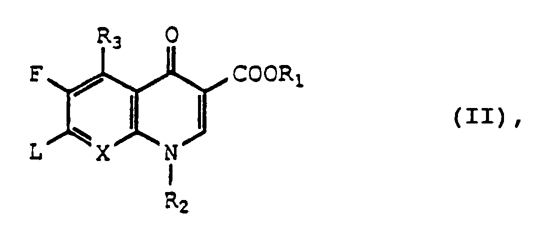
wherein R₅ and R₆ are each independently hydrogen and alkyl with 1 to 4 carbon atoms and n is an integer of from 0 to 4, a process of manufacture and pharmaceutical compositions useful as antibacterials.

## Description

EP-A-0 326 891 discloses compounds of formula I 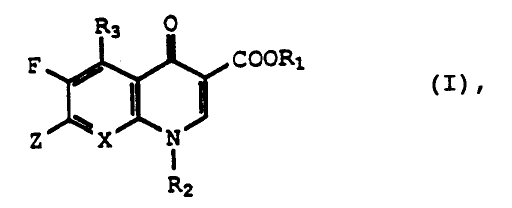
or a pharmaceutically acceptable acid addition or base salt thereof wherein Z is 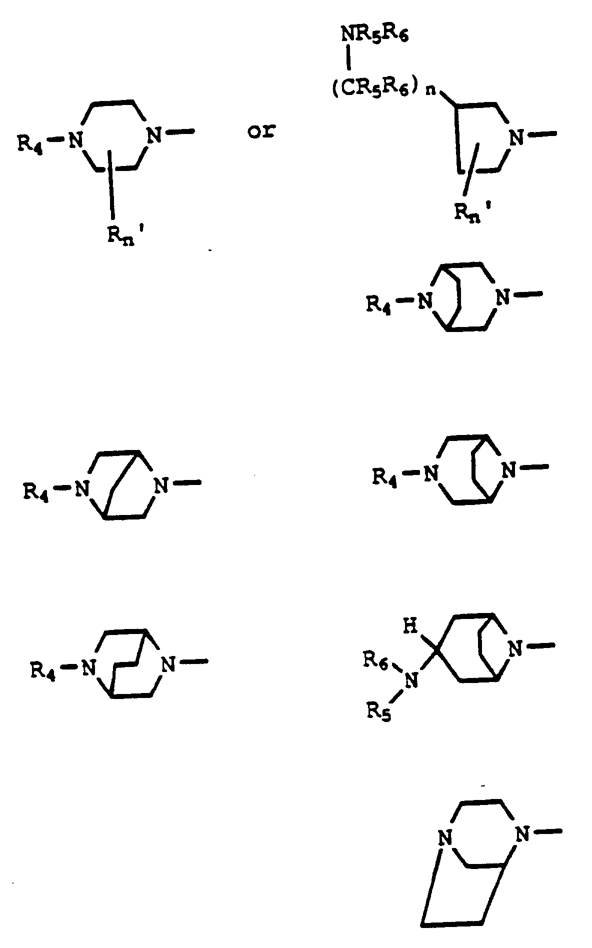
wherein R₄ is hydrogen, alkyl of from one to four carbon atoms, a cycloalkyl of from three to six carbon atoms, R' is hydrogen, hydroxyl, alkyl of from one to four carbon atoms, phenyl or phenyl substituted by halogen, alkyl or alkoxy, n is an integer of from 0 to 4, R₅ and R₆ are each independently hydrogen, lower alkyl or cycloalkyl;
X is CH, CF, CCl, CBr, N, CCF₃, CNH₂, CNO₂, CR, or COR' wherein R is lower alkyl and R' is hydrogen or lower alkyl,;
R₃ is lower straight, branched, or cyclic alkyl of from one to three carbon atoms;
R₂ is alkyl of from one to four carbon atoms, vinyl, haloalkyl, hydroxyalkyl of from two to four carbon atoms, cycloalkyl of from three to six carbon atoms, phenyl or phenyl substituted by halogen, alkyl, NH₂ or OH; and
R₁ is hydrogen, alkyl of from one to six carbon atoms, or a cation.

Another aspect of EP-A- 0 326 891 is a process of preparing compounds of Formula I 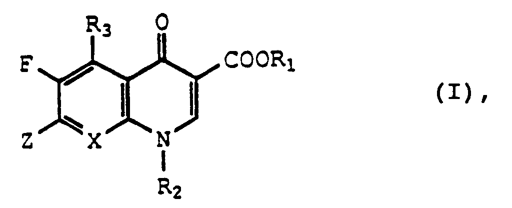
wherein R₁, R₂, R₃, R₄, R₅, R₆, Z, n, R', and X are as defined above which comprises reacting a compound of Formula II 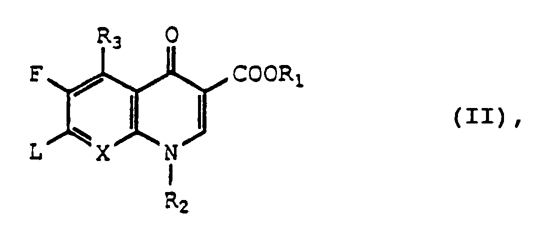
with an amine corresponding to the group Z wherein all of the above terms are as defined above in Formula I and L is a leaving group which may preferably be fluorine or chlorine.

The compounds of Formula I are effective antibacterial agents.

It has now been found that a small group of compounds of Formula I wherein X is nitrogen are especially valuable and show an especially desirable profile of activity. The selected compounds of the present invention belong to a group of naphthyridine-derivatives represented by the Formula III 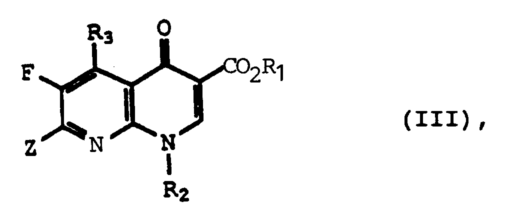
wherein R₁, R₂, R₃ and Z have the above described meaning. This selected group is characterized by formula IV 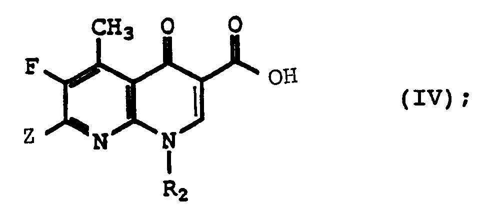
wherein R₂ is cyclopropyl or 2,4-difluorophenyl and Z is a group 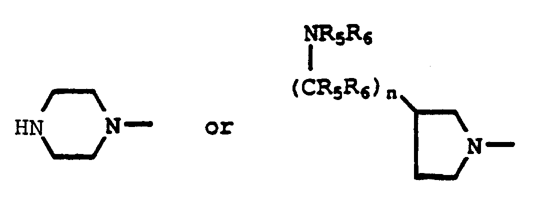
wherein R₅ and R₆ are each independently hydrogen and alkyl with 1 to 4 carbon atoms and n is an integer of from 0 to 4.

The new compounds are prepared by the following process, which is also described in EP-A-0 326 891.

The process comprises the steps:
(a) reacting a compound of formula 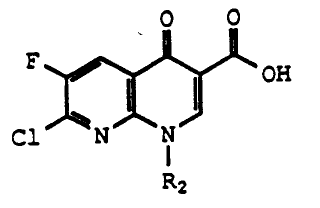 with oxalyl chloride and dimethylformamide and quenching with alcohol to produce the corresponding ester 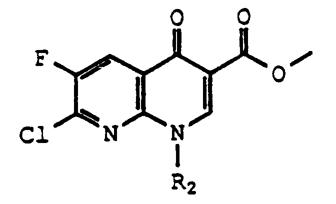
(b) reducing the double bond to produce a compound of formula 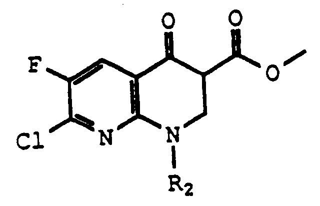
(c) treating the compound from step (b) with a base, then methyl iodide to produce the alkylated compound 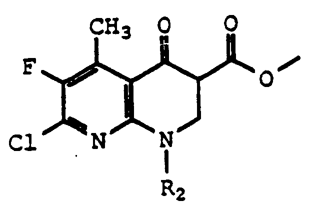
(d) reintroducing the double bond and reacting the resulting naphthyridine with the desired amine by known means.

The invention also includes a pharmaceutical composition which comprises an antibacterially effective amount of a selected compound having structural Formula IV and the pharmaceutically acceptable salts thereof in combination with a pharmaceutically acceptable carrier.

The invention further includes a process of preparing a pharmaceutical composition of a selected compound having structural Formula IV by incorporating said compound in a pharmaceutically acceptable carrier.

The invention further comprises the use of a selected compound according to Formula IV for the preparation of an antibacterial active pharmaceutical composition.

The following Schemes illustrate a synthesis of 5-alkyl naphthyridine-derivatives III; resp. IV.

### SCHEME I

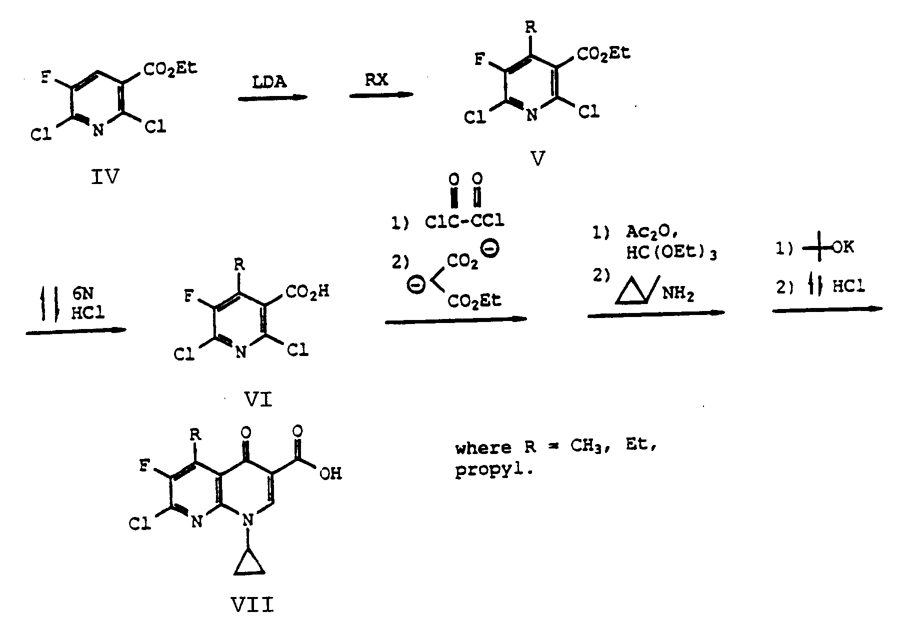

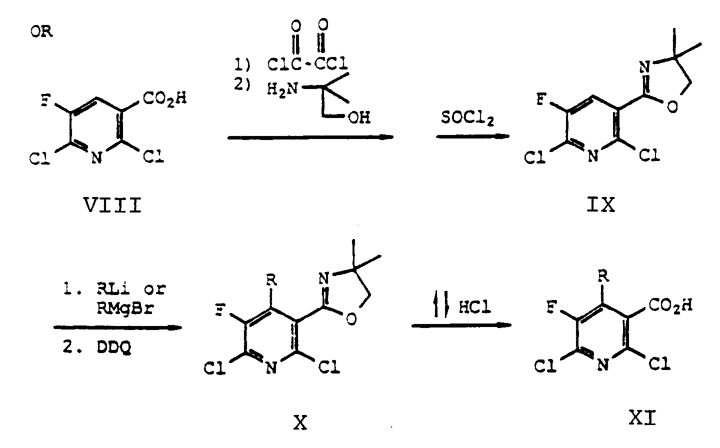

In Scheme I above pyridine ester IV (Chem. Pharm. Bull. 35 (1987), p 2280) is reacted with a base such as lithium diisopropylamide in THF at low temperature followed by an alkyl halide such as ethyl iodide or methyl iodide; hydrolysis of the ester in dilute acid affords compound VI.

Alternatively, ester IV can be hydrolyzed in dilute acid to give pyridine acid VIII which is, in turn, converted to the corresponding oxazoline in the usual manner. This oxazoline (Compound IX) is reacted with an alkyl lithium (such as methyl lithium), then rearomatized with DDQ or chloranil.
This sequence of reactions gives the alkyl-substituted pyridine X, which yields, upon acid hydrolysis, the necessary intermediate XI.

Compound XI can be elaborated to the 5-alkyl-7-chloro-1-cyclopropyl-6-fluoro-1,4-dihydro-4-oxo-1,8-naphthyridine-3-carboxylic acid in the usual manner.

### SCHEME II

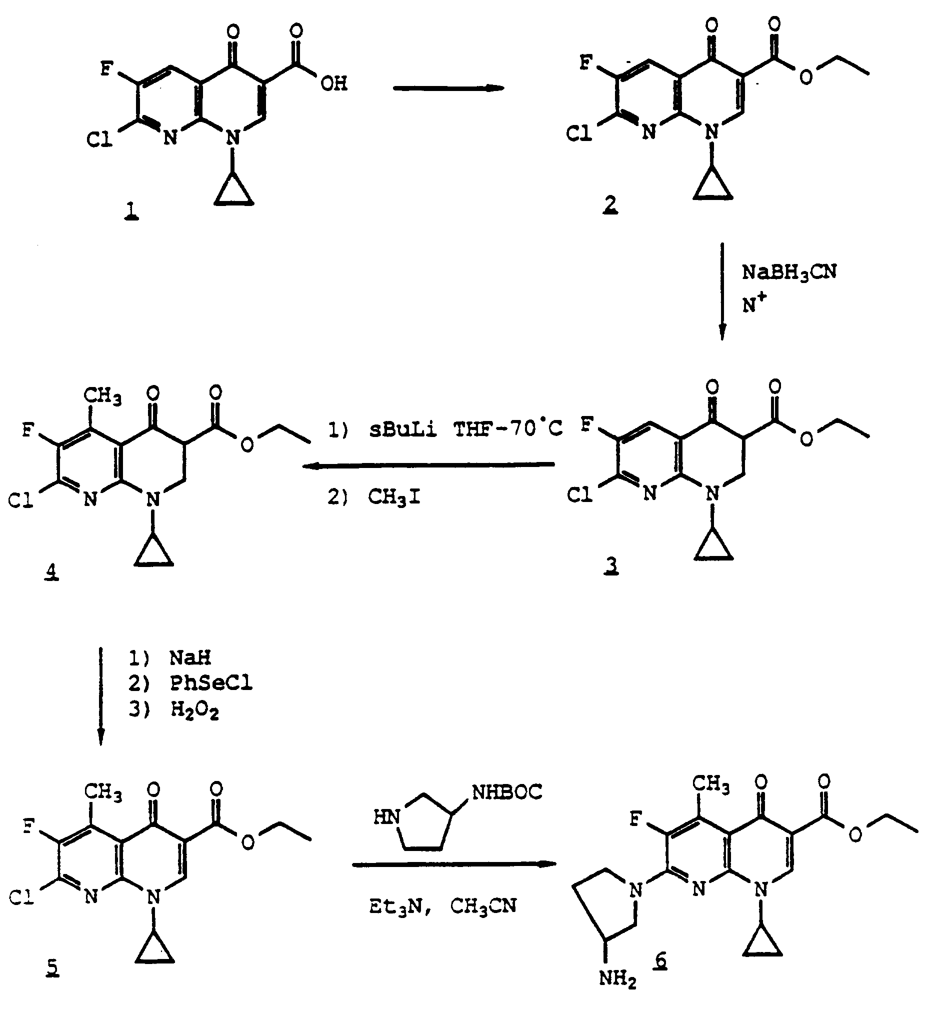

Also prepared by this method: 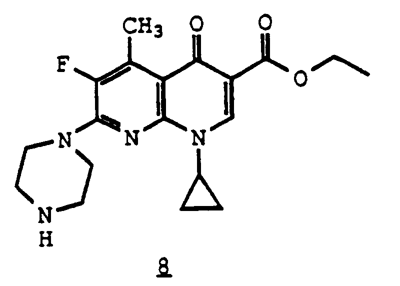
In Scheme II above, the known naphthyridine acid 1 (US Patent 4,663,457, 1987) is reacted with oxalyl chloride and DMF and then quenched with absolute ethanol to give ester 2. Reduction of the double bond is accomplished with sodium cyanoborohydride to afford compound 3, which is then treated with secbutyllithium at -78°C. The dianion is treated with methyl iodide to give the alkylated intermediate 4. The double bond is reintroduced in a series of steps: first, treatment with sodium hydride, followed by addition of phenylselenyl chloride and oxidation with hydrogen peroxide. The final ester 5 can then be reacted with a variety of amines the usual fashion.

### SCHEME III

Scheme III outlines an additional synthesis of 5-alkyl 1-substituted-1,8-naphthyridinecarboxylic acid derivatives. 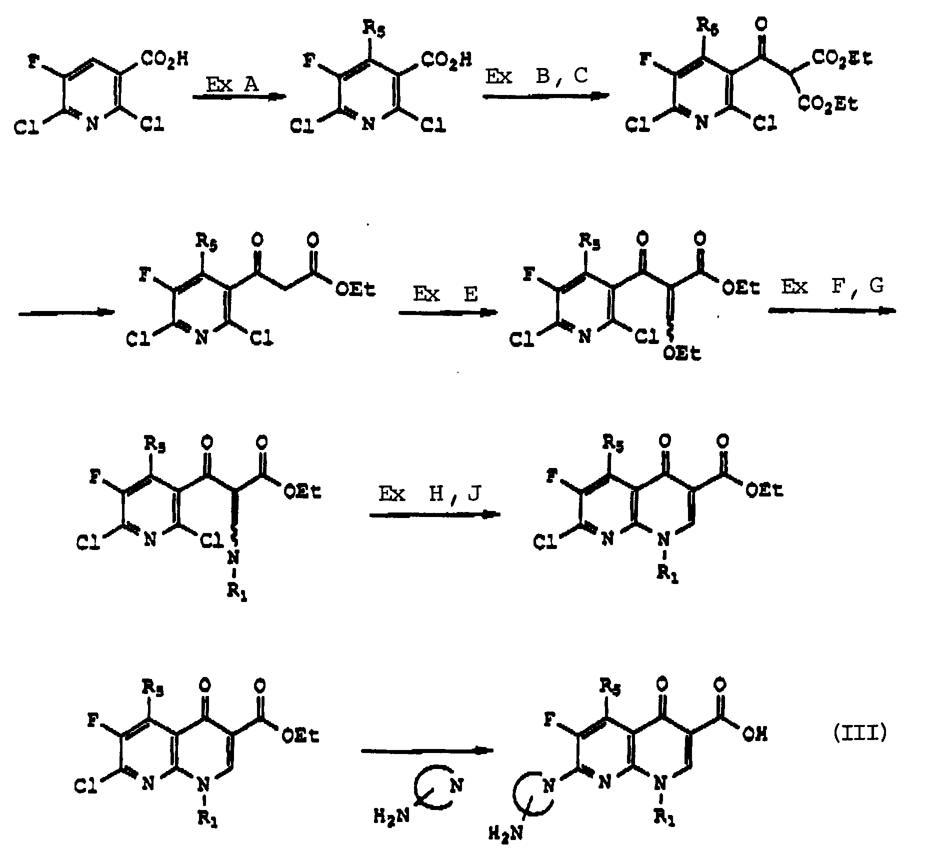
The introduction of the methyl group on the 1,8-naphthyridine substrate was accomplished by the formation of the di-anion of 2,6-dichloro-5-fluoro-nicotinic acid, preferably with lithium diisopropylamide in THF between -70°C and -50°C. The di-anion was quenched with iodomethane and the target acid 2,6-dichloro-5-fluoro-4-methylnicotinic acid is obtained after acidification. The remaining portion of the naphthyridine nucleus was elaborated using previously developed methodology.

The preferred selected compounds of formula IV are:
1. 7-(3-amino-1-pyrrolidinyl)-1-(2,4-difluorophenyl)-6-fluoro-1,4-dihydro-5-methyl-4-oxo-1,8-naphthyridine-3-carboxylic acid,
2. 3R,1'S-7-[3-(1-amino-ethyl)-1-pyrrolidinyl]-1-(2,4-difluorophenyl)-6-fluoro-1,4-dihydro-5-methyl-4-oxo-1,8-naphthyridine carboxylic acid,
3. 3R, 1'S-7-[3-(1-(N-methylamino)ethyl)-1-pyrrolidinyl]-1-(2,4-difluorophenyl)-6-fluoro-1,4-dihydro-5-methyl-4-oxo-1,8-naphthyridine-3-carboxylic acid,
4. 3R-7-[3-(1-amino-1-methylethylpyrrolidin-1-yl]-1-(2,4-difluorophenyl)-6-fluoro-1,4-dihydro-5-methyl-4-oxo-1,8-naphthyridine-3-carboxylic acid,
5. 3S-7-[3-amino-1-pyrrolidinyl]-1-(2,4-difluorophenyl) -6-fluoro-1,4-dihydro-5-methyl-4-oxo-1,8-naphthyridine-3-carboxylic acid,
6. 7-[(3-N-ethylaminomethyl)pyrrolidin-1-yl]-1-cyclopropyl -6-fluoro-1,4-dihydro-5-methyl-4-oxo-1,8-naphthyridine-3-carboxylic acid, and
7. 7-(3-aminomethylpyrrolidin-1-yl)-1-cyclopropyl-6-fluoro-1,4-dihydro-5-methyl-4-oxo-1,8-naphthyridine carboxylic acid.

The compounds are prepared according to the following Examples:

### I. Starting materials:

### Example A

### 2,6-Dichloro-4-methyl-5-fluoronicotinic acid

To a solution of 0.516 moles of lithio-diisopropyl amine in 330 mL hexane and 200 mL tetrahydrofuran, cooled to -70°C, was added dropwise a solution of 40g (0.229 moles) 2,6-dichloro-5-fluoronicotinic acid in 600 mL tetrahydrofuran, over a 1-hour period. The reaction mixture was stirred at -70°C for 1 hour, then warmed to -60°C for 1.5 hours at which time a light colored precipitate formed. The slurry was briefly warmed to -50°C, then recooled to -70°C, at which point a solution of 84 mL (1.34 moles) methyl iodide in 90 mL tetrahydrofuran was added dropwise. After the addition was complete, the reaction mixture was allowed to slowly warm to -20°C over approximately 1 hour. The solution was acidified with dilute HC1 pH 1-2 diluted with diethyl ether and allowed to warm to room temperature. The aqueous layer was extracted twice with ether. The combined ether washes were washed with H₂O, dried with M_{g}SO₄, and evaporated in vacuo to yield a crude solid. The crude material was recrystallized from toluene to yield 31.7 g of the title compound.

### Example B

### 2,6-Dichloro-4-methyl-5-fluoronicotinic acid chloride

A slurry of 30.8 g (0.147 moles) of 2,6-dichloro-4-methyl-5-fluoronicotinic acid in 200 mL of dichloromethane, containing a catalytic amount of dimethyl formamide, was treated dropwise over a 1-hour period with 19.2 mL (0.22 moles) oxalyl chloride. After 1 hour, the homogenous solution was evaporated in vacuo, diluted with an equal volume of dichloromethane, and evaporated in vacuo to yield 31.6 g of the title compound as an oil.

### Example C

### Ethyl 3-(2,6-dichloro-4-methyl-5-fluoropyridinyl)-3-oxo-2-carboxyethylpropanoate

A suspension of 4.5 g of magnesium turning (0.188 moles) in 5 mL of carbon tetrachloride and 11 mL of ethanol was treated dropwise with 30.1 g (0.168 moles) of diethyl malonate in 20 mL of ethanol at such a rate to maintain a gentle reflux. The mixture was heated et reflux for an additional 1.5 hours. The slurry was cooled to room temperature and diluted with 150 mL of diethyl ether. The resulting solution was cooled to 0°C and treated dropwise with 31.6 g of the product from Examples SS in 60 mL of ether. Mixing continued for 15 minutes after the completion of the addition and the mixture was stirred at room temperature for an additional hour. The mixture was poured into 250 mL of H₂O/10 mL of concentrated H₂SO₄. The mixture was extracted twice with ether. The combined ether extracts were washed with H₂O, dried over MgSO₄, and evaporated in vacuo to yield 56.4 g of the title compound.

### Example D

### Ethyl 3-(2,6-dichloro-4-methyl-5-fluoropyridinyl)-3-oxopropanoate

A suspension of 50.6 9 (0.147 moles) of the product from Example TT in 350 mL of H₂O containing 11.0 g p-toluenesulfonic acid hydrate was heated to reflux for a total of 3 hours. The suspension was cooled and diluted with diethyl ether. The ethereal layer was extracted with saturated NaHCO₃, water, and then dried with MgSO₄. After evaporation of the ether, 33.6 g (78%) of the title compound was obtained as an oil.

### Example E

### Ethyl 3-(2,6-dichloro-4-methyl-3-fluoro-5-pyridinyl)-3-oxo-2-ethoxymethylenepropanoate

A mixture of 33.6 g (0.114 moles) of the keto ester from Example D, 200 mL of acetic anhydride, and 30 mL (0.171 mole) of triethyl orthoformate was refluxed with external heat for a total of 4 hours. The mixture was concentrated in vacuo, redissolved in an equal volume of ethanol, and again concentrated in vacuo to yield 38.5 g of the title compound as an oil.

### Example F

### Ethyl 2-(2,4-difluorophenylaminomethylene)-3-(2,6-dichloro-3-fluoro-4-methyl-5-pyridyl)-3-oxopropanoate

A solution of 12.0 g (34 moles) of the product from Example E, 30 mL of ethanol, and 10 mL of hexane was cooled to 0°C and treated dropwise with 3.5 mL of 2,4-difluoroaniline dissolved in 5 mL of ethanol and 5 mL of hexane. After mixing for 1 hour at 0°C, a precipitate began to form. The suspension was allowed to mix at room temperature for 2 hours, during which time 10 mL of hexane was added to facilitate mixing. Filtration of the resulting solid afforded 3.6 g of the title compound. The mother liquors were purified on a silica gel column (hexane/ethyl acetate 80/20) to yield an additional 4.5 g of the title compound.

### Example G

### Ethyl 7-chloro-1-(2,4-difluorophenyl)-1,4-dihydro-5-methyl-1,8-naphthyridine-3-carboxylate

A solution of 8.0 g (18.8 mmol) of the product from Example F in 100 mL of 1,4-dioxane was treated portionwise with 0.8 g (20.7 mmol) of 60% NaH (oil suspension). The mixture was stirred at room temperature for 1.5 hours, then warmed to 50°C for 0.5 hours. The mixture was cooled and quenched with dilute hydrochloric acid. After evaporation, the residue was dissolved in ethyl acetate and washed with H₂O, dried with MgSO₄ and concentrated in vacuo. The residue was purified by column chromatography (silicon gel dichloromethane/ethanol 95:5) to yield 2.94 g of the title compound.

### Example H

### Ethyl 2-((cyclopropyl)aminomethylene)-3-(2,6-dichloro-3-fluoro-4-methyl-5-pyridyl)-3-oxopropanoate

A solution of 26.4 g (75 mmol) of the product in Example E in 65 mL of ethanol and 15 mL of hexane, cooled to 0°C, was treated dropwise with a solution of 4.7 g (83 mmole) of cyclopropyl amine in 10 mL of ethanol and 5 mL of hexane. The solution was stirred at 0°C for 1 hour and stirred for 2 hours it room temperature. After evaporation of the solvents in vacuo, the residue was purified by column chromatography (silica gel hexane-ethyl acetate 8:2) to afford 13.3 g of the title compound.

### Example I

### Ethyl 7-chloro-1-cyclopropyl-1,4-dihydro-6-fluoro-5-methyl-4-oxo-1,8-naphthyridine-3-carboxylate

A solution of 13.1 g (36.3 mmole) of the product from Example H is 300 mL of 1,4-dioxane is treated portionwise with 1.5 g (38.1 mmole) 60% NaH (oil suspension) at room temperature. The mixture was stirred for 1 hour at room temperature, then heated to 50°C for 0.5 hours. The cooled solution was quenched with dilute hydrochloric acid and the solvent evaporated in vacuo. The residue was dissolved in ethyl acetate, washed with H₂O, dried with MgSO₄, and concentrated in vacuo. The crude residue was purified by column chromatography (silica gel methylene chloride/ethanol 95:5) to afford 10.8 g of the title compound, mp 170-171°C. The product is identical to the material prepared in Example II.

### II. Compounds of Formula IV:

### Example 1

### 7-(3-Amino-1-pyrrolidinyl)-1-(2,4-difluorophenyl)-6-fluoro-1,4-dihydro-5-methyl-4-oxo-1,8-naphthyridine-3-carboxylic acid

A mixture of 0.7 g (2 mmole) of the product from Example G, 0.6 mL (4 mmole) triethylamine, 0.5 g (2.5 mmole) 3-(t-butoxycarbonylamino)pyrrolidine and 15 mL acetonitrile was refluxed for 1 hour. The mixture was concentrated in vacuo and purified by column chromatography (silica gel dichloromethane/ethanol 97:3) to yield 0.6 g of the fully protected title compound. The product was dissolved in 15 mL acetic acid and 15 mL 6N HCl and heated to reflux for 10 hours. The solvent was removed by evaporation in vacuo and the residue was dissolved in H₂O with dilute NaOH. The filtered solution was neutralized to a pH 7 with dilute HCl. The product was collected by filtration and washed with H₂O, acetonitrile, and ether to afford 0.36 g of the title compound, mp 260°C.

| Analyses for C₂₀H₁₇H₄O₃F₃ · 1.5 H₂O: | | | |
|---|---|---|---|
| Calcd.: | C, 53.92; | H, 4.49; | N, 12.57; |
| Found: | C, 54.10; | H, 4.27; | H, 12.20. |

### Example 2

### 3R,1'S-7-[3-(1-Aminoethyl)-1-pyrrolidinyl]-1-(2,4-difluorophenyl)-6-fluoro-1,4-dihydro-5-methyl-4-oxo-1,8-naphthyridine carboxylic acid

A solution of 0.67 g (2.9 mmole) of the compound from Example G, 0.8 g (3.1 mmole) 3R,1'S-3-(1-(t-butoxycarbonyl)aminoethyl)pyrrolidine, 1.2 mL (8.6 mmole) triethylamine and 15 mL acetonitrile was heated to reflux for 1 hour. The mixture was concentrated in vacuo and the residue was purified by column chromatography (silica gel dichloromethane/ethanol 97:3) to yield 0.9 g of the fully protected title compound. The compound was refluxed in a solution of 15 mL acetic acid and 15 mL of 6N hydrochloric acid for 8 hours and evaporated in vacuo. The residue was dissolved in H₂O with dilute NaOH, filtered, and neutralized with dilute hydrochloric acid to a final pH of 7. The product was collected by filtration, washed with H₂O, dissolved in 6N HCl, diluted with H₂O, and concentrated to dryness. Recrystallization from isopropyl alcohol/diethyl ether afforded 0.37 g of the title compound as the HCl salt, mp 297-299°C.

| Analysis for C₂₂H₂₁N₄O₆F₃ · 0.3 H₂O · 1.0 HCl: | | | |
|---|---|---|---|
| Calcd.: | C, 54.11; | H, 4.66; | N, 11.46; |
| Found: | C, 54.01; | H, 4.68; | N, 11.40. |

### Example 3

### 3R,1'S-7-[3-(1-(N-Methylamino)ethyl)-1-pyrrolidinyl]-1-(2,4-difluorophenyl)-6-fluoro-1,4-dihydro-5-methyl-4-oxo-1,8-naphthyridine-3-carboxylic acid

In 15 mL acetonitrile, the product from Example G, 0.5 g (1.5 mmole) along with 0.65 mL (4.6 mmole) triethylamine, and 0.45 g (1.7 mmole) 3R,1'S-3-[1-(t-butoxycarbonyl)methylaminoethyl]pyrrolidine was heated to reflux for 1 hour. After removal of the solvents in vacuo, the residue was purified by column chromatography (silica gel dichloromethane/ethanol 95:9) to yield 0.6 g of the fully protected title compound. Deprotection was achieved by refluxing the above compound in a mixture of 15 mL acetic acid and 15 mL 6N hydrochloric acid for 28 hours. The solvents were removed in vacuo and the product dissolved in H₂O using dilute NaOH. The filtered solution was neutralized with dilute hydrochloric acid to a pH 7. The product was collected by filtration and washed with H₂O. The solid was dissolved in concentrated hydrochloric acid, diluted with H₂O, and concentrated in vacuo. The residue was recrystallised from isopropanol/diethyl ether to yield 0.3 g of the title compound as the hydrochloric salt, mp 280°C dec.

| Analyzed for C₂₃H₂₃N₄O₃F₃ · 0.2 H₂O · 1.0 HCl: | | | |
|---|---|---|---|
| Calcd.: | C, 55.18; | H, 4.91; | N, 11.19; |
| Found: | C, 55.05; | H, 4.71; | N, 10.93. |

### Example 4

### 3R-7-[3-(1-Amino-1-methylethylpyrrolidin-1-yl]-1-(2,4-difluorophenyl)-6-fluoro-1,4-dihydro-5-methyl-4-oxo-1,8-naphthyridine-3-carboxylic acid

A mixture of 0.33 g (1 mmole) of the product from Example G, 0.30 g (1.1 mmole) 3R-(1-(t-butoxycarbonylamino)-1-methylethyl)pyrrolidine hydrochloride, 0.42 mL (7 mmole) triethylamine and 20 mL acetonitrile was heated to reflux for a total of 3 hours. The crude mixture was concentrated in vacuo, dissolved in 20 mL acetic acid/20 mL 6N hydrochloric acid, and heated to reflux for 28 hours. After removal of the solvents in vacuo, the crude product was dissolved in H₂O with dilute NaOH, filtered, and the solution was neutralized with dilute HCl to a final pH 6.8. The product was collected by filtration and washed with H₂O. The solid was dissolved in concentrated hydrochloric acid, diluted with H₂O and evaporated to dryness. Recrystallization from isopropyl alcohol/ether afforded 0.23 g of the title compound as the HCl salt, mp 230-233°C.

| Analyzed for C₂₃H₂₃N₄O₃F₃ · 0.9 H₂O · 1.0 HCl: | | | |
|---|---|---|---|
| Calcd.: | C, 53.83; | H, 5.06; | N, 10.91; |
| Found | C, 53.83; | H, 5.23; | N, 10.61. |

### Example 5

### 3S-7-[3-Amino-1-pyrrolidinyl]-1-[2,4-difluorophenyl)-6-fluoro-1,4-dihydro-5-methyl-4-oxo-1,8-naphthyridine-3-carboxylic acid

A mixture of 0.80 g (2.2 mmole) of the product of Example G, 0.53 g (2.8 mmole) of S-3-(t-butoxycarbonyl)aminopyrrolidine, 0.9 ml (6.4 mmole) triethyl amine, and 20 mL acetonitrile was heated to reflux for 3 hours. After concentration in vacuo the residue was purified by column chromatography (silica gel dichloromethane/ethanol 95:5) to yield 0.85 g of the fully protected title compound. The compound was heated to reflux in a solution of 20 mL acetic acid and 20 mL 6N hydrochloric acid for 16 hours. After concentration in vacuo the product was dissolved in H₂O with dilute aqueous NaOH, filtered, and the solution was neutralized with dilute hydrochloric acid to a final pH of 7. The product was collected by filtration and washed with H₂O. The solid was dissolved in concentrated hydrochloric acid, diluted with H₂O, and evaporated to dryness.
Recrystallization from isopropyl alcohol/ether afforded 0.57 g of the title compound as the HCl salt, mp 210-211°C.

### Example 6

### 7-[(3-N-Ethylaminomethyl)pyrrolidin-1-yl]-1-cyclopropyl-6-fluoro-1,4-dihydro-5-methyl-4-oxo-1,8-naphthyridine-3-carboxylic acid

A solution of 0.7 g (2 mmole) of the product from Example I, 0.4 g (3.1 mmole) of 3-N-ethylpyrrolidinemethanamine, 0.85 mL (6.1 mmole) triethylamine, and 20 mL acetonitrile was heated to reflux for a total of 8 hours. After concentration in vacuo, the compound was partially purified by column chromatography (silica gel-dichloromethane/ethanol/triethylamine 94:3:1) to yield 1.1 g. This residue was dissolved in 20 mL methanol/5 mL H₂O and treated with 4 mL of 1N NaOH. After stirring at room temperature for 3.5 hours, the methanol was removed in vacuo, and the mixture diluted with H₂O, and the solution was extracted once with dichloromethane. After filtration, the basic aqueous layer was neutralized with dilute hydrochloric acid to a final pH of 7. The product was collected by filtration and washed with H₂O, ether, and dried to afford 0.55 g of the title compound, mp 157-66°C.

| Analyzed for C₂₀H₂₅N₄O₃F · 0.7 H₂O: | | | |
|---|---|---|---|
| Calcd.: | C, 59.81; | H, 6.58; | N, 13.97; |
| Found: | C, 59.73; | H, 6.51; | N, 14.08. |

### Example 7

### 7-(3-Aminomethylpyrrolidin-1-yl)-1-cyclopropyl-6-fluoro-1,4-dihydro-5-methyl-4-oxo-1,8-naphthyridine carboxylic acid

A mixture of the product of Example I, 0.7 g (2 mmole), 0.85 mL (6 mmole) triethylamine, 0.48 g (2.4 mmole) 3-(t-butoxycarbonyl)aminomethylpyrrolidine and 30 mL acetonitrile was heated to reflux for a total of 4 hours. The solvent was removed in vacuo and the crude residue was heated to reflux in a solution of 15 ml acetic acid/15 mL 6N HCl for a total of 24 hours. The solvent was removed in vacuo and the residue was dissolved in H₂O with dilute NaOH. The filtered solution was neutralized with dilute hydrochloric acid to a final pH of 7. The precipitate formed was collected by filtration to afford 0.24 g of the title compound, mp >285°C.

## Claims

1. A compound of formula IV 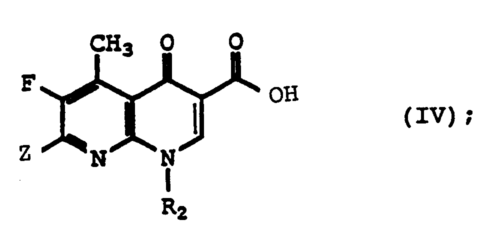 wherein R₂ is cyclopropyl or 2,4-difluorophenyl and Z is a group 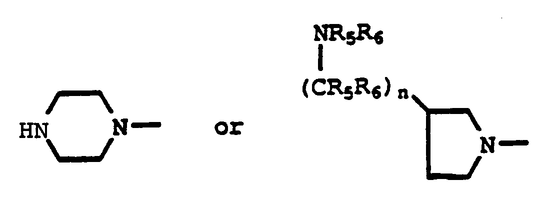 wherein R₅ and R₆ are each independently hydrogen and alkyl with 1 to 4 carbon atoms and n is an integer of from 0 to 4.

2. A compound according to Claim 1 selected from:
7-(3-amino-1-pyrrolidinyl)-1-(2,4-difluorophenyl)-6-fluoro-1,4-dihydro-5-methyl-4-oxo-1,8-naphthyridine-3-carboxylic acid,
3R,1'S-7-[3-(1-amino-ethyl)-1-pyrrolidinyl]-1-(2,4-difluorophenyl)-6-fluoro-1,4-dihydro-5-methyl-4-oxo-1,8-naphthyridine carboxylic acid,
3R,1'S-7-[3-(1-(N-methylamino)ethyl)-1-pyrrolidinyl]-1-(2,4-difluorophenyl) -6-fluoro-1,4-dihydro-5-methyl-4-oxo-1,8-naphthyridine-3-carboxylic acid,
3R-7-[3-(1-amino-1-methylethylpyrrolidin-1-yl]-1-(2,4-difluorophenyl)-6-fluoro-1,4-dihydro-5-methyl-4-oxo-1,8-naphthyridine-3-carboxylic acid,
3S-7-[3-amino-1-pyrrolidinyl]-1-(2,4-difluorophenyl)-6-fluoro-1,4-dihydro-5-methyl-4-oxo-1,8-naphthyridine-3-carboxylic acid,
7-[(3-N-ethylaminomethyl)pyrrolidin-1-yl] -1-cyclopropyl-6-fluoro-1,4-dihydro-5-methyl-4-oxo-1,8-naphthyridine-3-carboxylic acid, and
7-(3-aminomethylpyrrolidin-1-yl) -1-cyclopropyl-6-fluoro-1,4-dihydro-5-methyl-4-oxo-1,8-naphthyridine carboxylic acid.

3. A process of preparing a compound of Formula IV 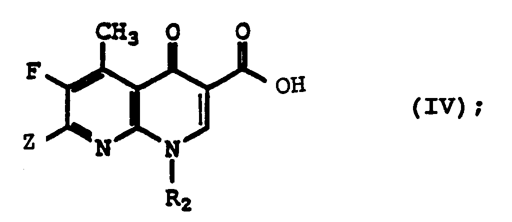 wherein R₂ is cyclopropyl or 2,4-difluorophenyl and Z is a group 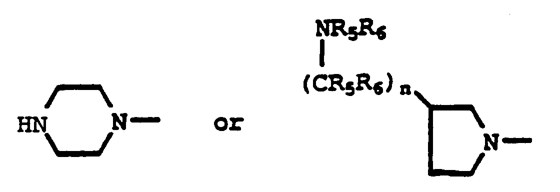 wherein R₅ and R₆ are each independently hydrogen and alkyl with 1 to 4 carbon atoms and n is an integer of from 0 to 4 characterized by
(a) reacting a compound of formula 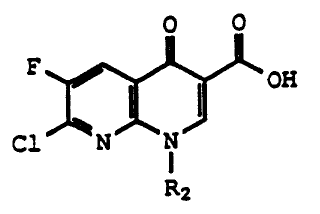 with oxalyl chloride and dimethylformamide and quenching with alcohol to produce the corresponding ester 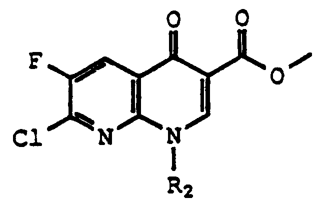
(b) reducing the double bond to produce a compound of formula 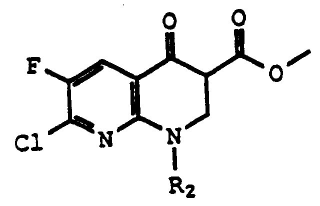
(c) treating the compound from step (b) with a base, then methyl iodide to produce the alkylated compound 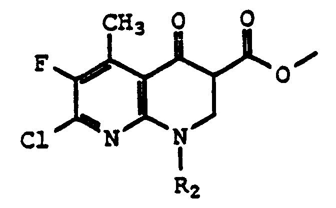
(d) reintroducing the double bond and reacting the resulting naphthyridine with the desired amine by known means.

4. A process of Claim 3 preparing a compound named
7-(3-amino-1-pyrrolidinyl)-1-(2,4-difluorophenyl)-6-fluoro-1,4-dihydro-5-methyl-4-oxo-1,8-naphthyridine-3-carboxylic acid,
3R,1'S-7-[3-(1-amino-ethyl)-1-pyrrolidinyl]-1-(2,4-difluorophenyl)-6-fluoro-1,4-dihydro-5-methyl-4-oxo-1,8-naphthyridine carboxylic acid,
3R,1'S-7-[3-(1-(N-methylamino)ethyl)-1-pyrrolidinyl]-1-(2,4-difluorophenyl) -6-fluoro-1,4-dihydro-5-methyl-4-oxo-1,8-naphthyridine-3-carboxylic acid,
3R-7-[3-(1-amino-1-methylethylpyrrolidin-1-yl]-1-(2,4-difluorophenyl)-6-fluoro-1,4-dihydro-5-methyl-4-oxo-1,8-naphthyridine-3-carboxylic acid,
3S-7-[3-amino-1-pyrrolidinyl]-1-(2,4-difluorophenyl)-6-fluoro-1,4-dihydro-5-methyl-4-oxo-1,8-naphthyridine-3-carboxylic acid,
7-[(3-N-ethylaminomethyl)pyrrolidin-1-yl] -1-cyclopropyl-6-fluoro-1,4-dihydro-5-methyl-4-oxo-1,8-naphthyridine-3-carboxylic acid, and
7-(3-aminomethylpyrrolidin-1-yl) -1-cyclopropyl-6-fluoro-1,4-dihydro-5-methyl-4-oxo-1,8-naphthyridine carboxylic acid.

5. A pharmaceutical Composition consisting of a compound according to Claims 1 to 4 and a pharmaceutically acceptable carrier.

6. Process for manufacturing a pharmaceutical composition comprising a compound according to Claims 1 to 4 characterized by incorporating the compound in a pharmaceutically acceptable carrier.

7. Use of a compound according to Claims 1 to 4 for the preparation of an antibacterially active pharmaceutical composition.
